Europäisches Patentamt

**(19)** European Patent Office

Office européen des brevets

**(11)** **EP 0 628 320 B1**

**(12)** **EUROPEAN PATENT SPECIFICATION**

**(45)** Date of publication and mention
of the grant of the patent:
**11.08.1999 Bulletin 1999/32**

**(51)** Int. Cl.$^6$: **A61L 15/58**

**(21)** Application number: **94107631.7**

**(22)** Date of filing: **17.05.1994**

**(54)** **Medical adhesive tape and tape preparation**

Klebeband für medizinische Zwecke und Verfahren zu dessen Herstellung

Ruban adhésif à usage médical et son procédé de préparation

**(84)** Designated Contracting States:
**CH DE ES FR GB IT LI NL**

**(30)** Priority: **17.05.1993 JP 11451293**
**08.06.1993 JP 13731793**

**(43)** Date of publication of application:
**14.12.1994 Bulletin 1994/50**

**(73)** Proprietor:
**NITTO DENKO CORPORATION**
**Osaka (JP)**

**(72)** Inventors:
• **Higashio, Kazuhiro,**
**c/o Nitto Denko Corporation**
**Ibaraki-shi, Osaka (JP)**
• **Akemi, Hitoshi,**
**c/o Nitto Denko Corporation**
**Ibaraki-shi, Osaka (JP)**
• **Kinoshita, Takashi,**
**c/o Nitto Denko Corporation**
**Ibaraki-shi, Osaka (JP)**
• **Muraoka, Takateru,**
**c/o Nitto Denko Corporation**
**Ibaraki-shi, Osaka (JP)**
• **Otsuka, Saburo,**
**c/o Nitto Denko Corporation**
**Ibaraki-shi, Osaka (JP)**

**(74)** Representative:
**Grünecker, Kinkeldey,**
**Stockmair & Schwanhäusser**
**Anwaltssozietät**
**Maximilianstrasse 58**
**80538 München (DE)**

**(56)** References cited:
• **DATABASE WPI Section Ch, Week 9251, Derwent Publications Ltd., London, GB; Class A14, AN 92-419602 & JP-A-4 314 773 (NITTO DENKO CORP) 5 November 1992**
• **DATABASE WPI Section Ch, Week 8948, Derwent Publications Ltd., London, GB; Class A81, AN 89-352273 & JP-A-1 263 182 (NITTO DENKO CORP) 19 October 1989**
• **DATABASE WPI Section Ch, Week 7428, Derwent Publications Ltd., London, GB; Class A81, AN 74-50895V & JP-A-49 005 145 (DAINIPPON INK & CHEM INC) 17 January 1974**
• **DATABASE WPI Section Ch, Week 9211, Derwent Publications Ltd., London, GB; Class A81, AN 92-086203 & JP-A-4 031 477 (NITTO DENKO CORP) 3 February 1992**
• **DATABASE WPI Section Ch, Week 8626, Derwent Publications Ltd., London, GB; Class A81, AN 86-167243 & JP-A-61 101 584 (NITTO ELECTRIC IND KK) 20 May 1986**
• **DATABASE WPI Section Ch, Week 8713, Derwent Publications Ltd., London, GB; Class A96, AN 87-091482 & JP-A-62 042 920 (NITTO ELECTRIC IND KK) 24 February 1987**
• **PATENT ABSTRACTS OF JAPAN vol. 017, no. 478 (C-1104) 31 August 1993 & JP-A-05 117 613 (NITTO DENKO CORP) 14 May 1993**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

FIELD OF THE INVENTION

The present invention relates to a medical adhesive tape used for protecting a skin surface by adhering the same onto the skin surface and also to a tape preparation for continuously administering percutaneous absorption-type drugs into a living body through the skin by incorporating the percutaneous absorption-type drugs in a pressure-sensitive adhesive layer of the medical adhesive tape.

BACKGROUND OF THE INVENTION

Various types each comprising a nonwoven fabric or a plastic film having formed on one surface thereof a pressure-sensitive adhesive layer are proposed as a medical adhesive tape for protecting an injured portion of a skin surface and a tape preparation for percutaneous absorption for administering drugs into a living body through the skin surface. Usually, the medical adhesive tape, etc., using such a pressure-sensitive adhesive is required to have a skin adhesive force in a certain extent for preventing the adhesive from falling off from the skin surface to which the adhesive tape is applied. However, as the skin adhesive force becomes larger, a physical stimulative property at releasing the adhesive tape from a skin surface becomes larger to cause a pain or the release of a keratin layer at releasing the adhesive tape, whereby there is a possibility to give a pain to a user by giving an unnecessary stimulation or injury to the skin surface.

Accordingly, in the case of developing the medical adhesive tapes and tape preparations which are applied to a skin surface, the development of a material which has a good tackiness or adhesion to a skin surface and an excellent skin adhesive force which does not give a physical stimulation at releasing it from a skin surface and does not injure the skin surface has been desired.

Thus, as a result of various investigations to overcome the above problems, the inventors previously found that the skin stimulative property could be greatly reduced by using a pressure-sensitive adhesive layer which causes a specific shear deformation when a shearing stress is applied, and proposed the pressure-sensitive adhesive layer as described in JP-A-5-64460 (the term "JP-A" as used herein means an "unexamined published Japanese patent application").

Thereafter, as a result of further investigations, the inventors have found that by designing the pressure-sensitive adhesive layer such that the layer has the creep characteristics that the permanent strain formed in the pressure-sensitive adhesive layer and the initial shear modulus at applying a shearing stress to the pressure-sensitive adhesive layer are reduced as small as possible, the pressure-sensitive adhesive layer gives a proper soft feeling during the application thereof to a skin surface and the skin stimulative property at releasing the pressure-sensitive adhesive layer from the skin surface can be reduced, and have accomplished the present invention based on this finding.

Furthermore, it has also been found that when a shearing elastic coefficient (G) and a retarding time ($\tau$) in the case of simply applying a deformation strain of the pressure-sensitive layer to the Voigt model are adjusted to the ranges of specific values, the pressure-sensitive adhesive layer is excellent in the adhesive property to a skin surface and the moderating and dispersing properties of a stress occurring at releasing the pressure-sensitive adhesive layer and further can maintain a delicate balance between the skin adhesion and the non-stimulative property.

SUMMARY OF THE INVENTION

Accordingly, one object of the present invention is to provide a medical adhesive tape comprising a flexible support having formed at least one surface thereof a hydrophobic pressure-sensitive adhesive layer, wherein when the pressure-sensitive adhesive layer is adhered to a bakelite plate, an initial shear modulus in the case of applying a load of 50 g/cm$^2$ to a shearing direction for 30 seconds is 1 N/cm$^2$ ($1 \times 10^5$ dyn/cm$^2$) or less and a permanent shearing strain formed in the pressure-sensitive adhesive layer in the case of removing the load after applying the load for 30 minutes is 30% or less of the shearing strain at removing the load.

In the particularly preferred embodiment of the medical adhesive tape of the present invention, when the deformation strain at applying a shearing stress is applied to the simple Voigt model, the deformation strain has the shearing elastic coefficient (G) and the retarding time ($\tau$) satisfying the values of the following formulae;

$$0.6 \text{ N/cm2} > G > 0.02 \text{ N/cm}^2 \ [6.0 \times 10^4 > G > 2.0 \times 10^3 \ (\text{dyn/cm}^2)] \ 30 > \tau \text{ (sec.)}$$

Another embodiment of the present invention is to provide a tape preparation comprising the above-described medical adhesive tape containing percutaneous absorption-type drugs in the pressure-sensitive adhesive layer.

BRIEF DESCRIPTION OF THE DRAWINGS

Figure 1 is a view explaining the measurement method of the shear modulus in the Examples,
Figure 2 is a schematic view explaining the Voigt model,
Figure 3 is graph showing the time-strain curve in the case of applying a definite stress by the Voigt model, and
Figure 4 is a side view explaining the deformation occurred at applying a stress to the pressure-sensitive adhesive layer of the medical adhesive tape and the tape preparation of the present invention.

DETAILED DESCRIPTION OF THE INVENTION

The present invention is described in detail below.

The medical adhesive tape and the tape preparation of the present invention each comprises a flexible support having formed on at least one surface thereof a hydrophobic pressure-sensitive adhesive layer having specific properties containing optional percutaneous absorption-type drugs. The pressure-sensitive adhesive layer may be directly formed on the surface of the support or may be indirectly formed thereon via a conventional undercoat as a means for improving an anchoring property.

The thickness of the support is usually from about 0.5 to 50 μm, and preferably from about 1 to 25 μm from the point of the handling property and the occlusive dressing technique effect (ODT effect).

There is no particular restriction on the material for the support so long as the material has a flexibility, but in the case of the tape preparation, it is preferred to select from the materials that the percutaneous absorption-type drugs contained in the pressure-sensitive adhesive layer do not decrease the content thereof by being lost from the back surface of the support, that is, these components (components contained in the pressure-sensitive adhesive layer, e.g., a pressure-sensitive adhesive, a drug and an organic liquid material) do not permeate through the material. Practically, the single film of polyester, nylon, Saran (trade name of Dow Chemical Co.), polyethylene, polypropylene, an ethylene-vinyl acetate copolymer, polyvinyl chloride, an ethylene-ethyl acrylate copolymer, polytetrafluoroethylene, surlyn, a metal foil, etc., or laminated films thereof can be used.

Of these materials, to improve the adhesion (anchoring property) between the support and the hydrophobic pressure-sensitive adhesive layer described hereinafter, it is preferred that the support has a laminate structure comprising a non-porous sheet of the above-described material and a porous sheet such as a paper, a woven fabric, a nonwoven fabric, etc., and the pressure-sensitive adhesive layer is formed on the porous sheet side. When a woven fabric or a nonwoven fabric is used as the porous sheet, the weight thereof per unit area is from 5 to 30 g/m$^2$, and preferably from 8 to 20 g/m$^2$ from the point of improving the anchoring property.

The hydrophobic pressure-sensitive adhesive layer formed on at least one surface of the support described above in the present invention does not comprise polyacrylic acid, a polyacrylate, gelatin, etc., having an affinity with water such as a so-called hydrogel as the base material, but comprises a polymer of a monomer having a hydrophobic property as the main component.

The polymer which is preferably used is a (meth)acrylic acid alkyl ester polymer obtained by polymerizing a (meth)acrylic acid alkyl ester which can relatively easily control the pressure-sensitive adhesive characteristics as the main component monomer. Further, it is a preferred means to crosslink the pressure-sensitive adhesive layer to render the layer hydrophobic.

In the present invention, in addition to the above-described (meth)acrylic acid alkyl ester polymer, other hydrophobic pressure-sensitive adhesive such as a silicone polymer, a vinyl ether polymer, a rubber polymer, etc., can be used, but in the case of using such a pressure-sensitive adhesive, since the pressure-sensitive adhesive is difficult to control such that it satisfies the creep characteristics in the present invention and also has a selectivity in the solubility and the releasing property of percutaneous absorption-type drugs, it is necessary to pay close attention to handling thereof.

The (meth)acrylic acid alkyl ester polymer used in the present invention is a polymer obtained by homopolymerizing a (meth)acrylic acid alkyl ester or obtained by copolymerizing a (meth)acrylic acid alkyl ester as a main component and a copolymerizable monomer. The polymer becomes the main base material for showing a skin adhesion.

As the (meth)acrylic acid alkyl ester, the ester having an alkyl group having an average carbon atom number of from 4 to 18 is preferred from the point of the pressure-sensitive adhesive characteristics at adhering to a skin. Specific examples of the alkyl ester are straight chain alkyl esters and branched alkyl esters, such as a butyl ester, a pentyl ester, a hexyl ester, a heptyl ester, an octyl ester, a nonyl ester, a decyl ester, an undecyl ester, a dodecyl ester, a tridecyl ester, etc. These (meth)acrylic acid alkyl esters can be used alone or as a mixture thereof.

The average carbon atom number used herein means the average of the carbon atom number of the alkyl group in the (meth)acrylic acid alkyl ester used for the polymerization reaction, and the present invention does not exclude a (meth)acrylic acid alkyl ester having an alkyl group having from 1 to 3 carbon atoms or an alkyl group having 19 or more carbon atoms.

Examples of the monomer copolymerizable with the (meth)acrylic acid alkyl ester are carboxyl group-containing monomers such as (meth)acrylic acid, itaconic acid, maleic acid, maleic anhydride, crotonic acid, etc.; sulfoxy group-containing monomers such as styrenesulfonic acid, allylsulfonic acid, sulfopropyl (meth)acrylate, (meth)acryloyloxynaphthalenesulfonic acid, acrylamidomethylpropanesulfonic acid, etc.; hydroxyl group-containing monomers such as (meth)acrylic acid hydroxyethyl ester, (meth)acrylic acid hydroxypropyl ester, etc.; amido group-containing monomers such as (meth)acrylamide, dimethyl(meth)acrylamide, N-butylacrylamide, N-methylol (meth)acrylamide, N-methylolpropane (meth)acrylamide, etc.; alkylaminoalkyl group-containing monomers such as (meth)acrylic acid aminoethyl ester, (meth)acrylic acid dimethylaminoethyl ester, (meth)acrylic acid tert-butylaminoethyl ester, etc.; (meth)acrylic acid alkoxyalkyl esters such as (meth)acrylic acid methoxyethyl ester, (meth)acrylic acid ethoxyethyl ester, etc.; alkoxy group(or having an oxide bond at the side chain)-containing (meth)acrylic acid esters such as (meth)acrylic acid tetrahydrofurfuryl ester, (meth)acrylic acid methoxyethylene glycol ester, (meth)acrylic acid methoxydiethylene glycol ester, (meth)acrylic acid methoxypolyethylene glycol ester, etc.; vinyl monomers having a heterocyclic ring, such as N-vinyl-2-pyrrolidone, methylvinyl pyrrolidone, vinylpyridine, vinylpiperidone, vinylpyrimidine, vinylpiperazine, vinylpyrazine, vinylpyrrole, vinylimidazole, vinylcaprolactam, vinyloxazole, vinylmorpholine, etc.; vinyl ester monomers such as vinyl acetate, vinyl propionate, etc.; (meth)acrylonitrile, etc.

These monomers can be used alone or as a mixture thereof for the copolymerization.

The preferred monomers in the above monomers are the carboxyl group-containing monomers such as (meth)acrylic acid, etc., and the hydroxyl group-containing monomers such as (meth)acrylic acid hydroxyethyl ester, etc.

These monomers used for the copolymerisation can be used to control a cohesive force of the pressure-sensitive adhesive layer and to control a solubility of a drug when the pressure-sensitive adhesive layer contains the drug. Further, the copolymerization amount of the monomer can be optionally selected in the range of 50% by weight or less, and preferably from 2 to 40% by weight, according to the purpose.

In the present invention, to obtain the specific creep characteristics, it is preferred to design such that the pressure-sensitive adhesive layer has a feeling as soft as possible and for the purpose, it is preferred to add an organic liquid material compatible to the hydrophobic pressure-sensitive adhesive layer. In this case, by the addition of the organic liquid material, the pressure-sensitive adhesive layer is plasticized to decrease a cohesive force, whereby there is a possibility to lose the desired creep characteristics. Accordingly, in such a case, it is preferred that the pressure-sensitive adhesive layer is subjected to a crosslinking treatment by an optional crosslinking means to form the pressure-sensitive adhesive layer having a crosslinked structure.

The organic liquid material is a material which is in a liquid state at room temperature and is compatible with the hydrophobic pressure-sensitive adhesive layer.

Examples of the organic liquid material are glycols such as ethylene glycol, diethylene glycol, triethylene glycol, propylene glycol, polyethylene glycol, polypropylene glycol, etc.; fats and oils such as an olive oil, a castor oil, squalene, lanolin, etc.; organic solvents such as ethyl acetate, ethanol, dimethyldecyl sulfoxide, methyloctyl sulfoxide, dimethyl sulfoxide, dimethyl formamide, dimethyl acetamide, dimethyl laurylamide, dodecyl pyrrolidone, N-methylpyrrolidone, isosorbitol, etc.; liquid surface active agents; plasticizers such as diisopropyl adipate, phthalic acid esters, diethyl sebacate, etc.; hydrocarbons such as fluid paraffin, etc.; fatty acid esters such as isopropyl myristate, isotridecyl myristate, diethyl sebacate, ethyl laurate, ethyl oleate, oleic acid, diisopropyl adipate, isopropyl palmitate, octyl palmitate, etc.; fatty acids for obtaining these fatty acid esters; and organic compounds which are in liquid state at room temperature, such as a glycerol ester, ethoxylated stearyl alcohol, 1,3-butanediol, etc.

These organic liquid materials can be used alone or as a mixture thereof. In these organic liquid materials, the fatty acid esters such as isopropyl myristate, diethyl sebacate, octyl palmitate, ethyl oleate, diethyl phthalate, diisopropyl adipate, etc.; the fatty acids such as monocaprylic acid, caprylic acid, oleic acid, etc.; and the surface active agents such as sorbitan monocaprylate, etc., can be preferably used from the points of non-stimulative property to a skin surface and the improvement of percutaneous absorption property of drugs in the case of the tape preparation.

The organic liquid material plasticizes the pressure-sensitive adhesive layer to give thereto a soft feeling and also by, if necessary, crosslinking the pressure-sensitive adhesive layer, the organic liquid material is held in the hydrophobic pressure-sensitive adhesive layer in a compatible state therewith to give proper creep characteristics. As the result, when the pressure-sensitive adhesive layer is released from a skin surface, the organic liquid material has a function to reduce the pain and the skin stimulative property caused by the skin adhesive force of the pressure-sensitive adhesive layer.

The content of the organic liquid material contained in the pressure-sensitive adhesive layer is from 15 to 75% by weight, and preferably from 25 to 65% by weight, from the points of reducing the skin stimulative property and giving excellent creep characteristics. If the content of the organic liquid material is less than 15% by weight, there is a tendency that a sufficient effect of reducing the skin stimulative property cannot be expected, while if the content is over 75% by weight, the cohesive force of the pressure-sensitive adhesive layer is decreased and the pressure-sensitive adhesive layer causes a cohesive failure at releasing the layer, whereby the skin stimulative property tends to become large.

It is preferred in the present invention to make the pressure-sensitive adhesive layer a crosslinked structure to increase the cohesive force of the pressure-sensitive adhesive layer, whereby the desired creep characteristics are liable to obtain. That is, by making the pressure-sensitive adhesive layer a crosslinked structure, even when after adhering the pressure-sensitive adhesive layer to a skin surface, a shearing stress is added from the outside, the cohesive failure in the inside of the pressure-sensitive adhesive layer does not occur and the pressure-sensitive adhesive layer becomes a viscoelastic material which does not cause a peeling in the laminate interface between the support and the pressure-sensitive adhesive layer and a peeling at the adhered interface to a skin surface to which the pressure-sensitive adhesive layer is adhered. In particular, when the organic liquid material is compounded with the pressure-sensitive adhesive layer, the layer is plasticized. Therefore, it is effective to apply a crosslinking treatment to the pressure-sensitive adhesive layer.

The crosslinking treatments which can be employed are a physical crosslinking treatment by a radiation irradiation such as an ultraviolet irradiation, an electron-ray irradiation, etc., and a chemical crosslinking treatment using a crosslinking agent such as polyisocyanate compounds, organic peroxides, organic metal salts, metal alcoholates, metal chelate compounds, melamine derivatives, polyfunctional compounds, etc.

In these crosslinking means, when the radiation irradiation or the organic peroxide is used, it sometimes happens that a degradation reaction of the drug occurs according to the kind of the drug used, and also when a high-reactive isocyanate, or a metal salt or an organic metal salt used for an ordinary crosslinking reaction is used, it sometimes happens that after compounding such a crosslinking agent, a viscosity increasing phenomenon of the solution occurs to reduce the workability.

A method of previously copolymerizing a polyfunctional monomer such as a diacrylate, etc., with the acrylic polymer as the pressure-sensitive adhesive may be considered, but in this case, there is a possibility that the solution viscosity is increased at coating.

Accordingly, of these crosslinking agents, tri-functional isocyanates, metal alcoholates comprising titanium or aluminum, or metal chelate compounds are suitable from the points of reactivity and handling property. These crosslinking agents do not cause the viscosity increasing phenomenon of solution before coating and drying, and thus are very excellent in workability.

The amount of the crosslinking agent added in this case to obtain the creep characteristics in the numeral range of the present invention is as follows. For example, when the pressure-sensitive adhesive layer is formed using an acrylic acid alkyl ester polymer, the amount of the crosslinking agent added is from about 0.01 to 2 parts by weight per 100 parts by weight of the acrylic acid alkyl ester polymer.

Even when the polymer to be crosslinked which forms the pressure-sensitive adhesive layer does not have a functional group which is reacted with the crosslinking agent, the polymer can be denatured into a structure capable of being crosslinked by applying an alkali treatment, etc., to the polymer to be crosslinked.

By containing percutaneous absorption-type drugs in the pressure-sensitive adhesive layer of the medical adhesive tape of the present invention, a tape preparation which is utilized for the treatment and prophylaxis of various diseases by percutaneously administering the drugs into a living body is obtained. The pressure-sensitive adhesive layer of the tape preparation contains percutaneous absorption-type drugs in a dissolved state or a dispersed state.

The drug contained can be optionally selected according to the treatment purpose of a disease. Examples of the drugs used for the tape preparation of the present invention are corticosteroids, analgetic anti-inflammatory agents, hypnotic sedatives, tranquilizers, antihypertensive agents, hypotensive diuretics, antibiotics, anesthetics, antibacterial agents, antimycois agents, vitamins, coronary vasodilator agents, antihistamic agents, antitussive agents, sex hormones, antidepressive agents, cerebral improving agents, antiemetic agents, antitumor agents, and living body drugs. These drugs can be used alone or, if necessary, as a mixture thereof.

The content of the drugs can be properly selected according to the kind of the drugs and the administration purpose, but is usually from 1 to 40% by weight, and preferably from about 2 to 30% by weight, in the pressure-sensitive adhesive layer. If the content of the drugs is less than 1% by weight, the release of the amount of the drugs effective for the treatment of a disease cannot expected, and if the content is over 40% by weight, the limit occurs for the treatment effect of a disease and also the use of such a large amount of the drugs is economically disadvantageous

The medical adhesive tape and the tape preparation of the present invention have the feature in the creep characteristics of the pressure-sensitive adhesive layer. That is, the pressure-sensitive adhesive layer has the creep characteristics that when the pressure-sensitive adhesive layer is adhered to a bakelite plate, the initial shear modulus in the case of applying a load of 50 g/cm$^2$ to the shearing direction for 30 seconds is 1 N/cm2 ($1 \times 10^5$ dyn/cm$^2$) or less and when the load is applied for 30 minutes and the load is then removed, the permanent shearing strain formed in the pressure-sensitive adhesive layer becomes 30% or less of the shearing strain at removing the load.

The initial shear modulus in the present invention is calculated from the shearing strain after passing 30 seconds since the application of the load as shown in the measurement method described hereinafter, and the permanent shearing strain in the present invention is the shear occurring at the adhered interface of the surface of a bakelite plate and the pressure-sensitive layer or in the case of occurring a flow in the pressure-sensitive adhesive layer when the pressure-sensitive adhesive layer is adhered to the bakelite plate and a shearing stress is then applied.

By that the pressure-sensitive adhesive layer has the creep characteristics described above, the pressure-sensitive adhesive layer is excellent in the cohesive force and the damage to the skin surface at releasing the pressure-sensitive adhesive layer from the skin surface can be reduced as small as possible.

In a more preferred embodiment, by applying a crosslinking treatment to the pressure-sensitive adhesive layer in the present invention, the pressure-sensitive adhesive layer becomes a viscoelastic material satisfying the simple Voigt model and in the test conditions as described below, the cohesive failure which is an independent viscosity factor other than the viscosity factor (dash pot) connected in parallel with an elastic factor (spring) can be excluded. In addition, in order to coincide with the simple Voigt model, it is a necessary condition in the present invention that in the test method described hereinafter, the deformation is completed within 1,000 seconds.

That is, if the interfacial breakdown at the adhesion interface between a material to be adhered and the pressure-sensitive adhesive layer occur, the movement is not stopped even if the deformation is finished, whereby the pressure-sensitive adhesive layer is measured such that as if the layer has the independent viscosity factor and causes a cohesive failure, and an erroneous judgement is made that the pressure-sensitive adhesive layer does not satisfy the conditions (the G value and the $\tau$ value) of the present invention.

Accordingly, it is preferred that by using the pressure-sensitive adhesive layer described above for the medical adhesive tape and the tape preparation of the present invention, the pressure-sensitive adhesive layer coincides with the simple Voigt model and has the shearing elastic coefficient (G) and the retarding time ($\tau$) satisfying the following values.

$$0.6 \text{ N/cm}^2 > G > 0.02 \text{ N/cm}^2 \ [6.0 \times 10^4 > G > 2.0 \times 10^3 \ (\text{dyn/cm}^2)] \ 30 > \tau \ (\text{sec.})$$

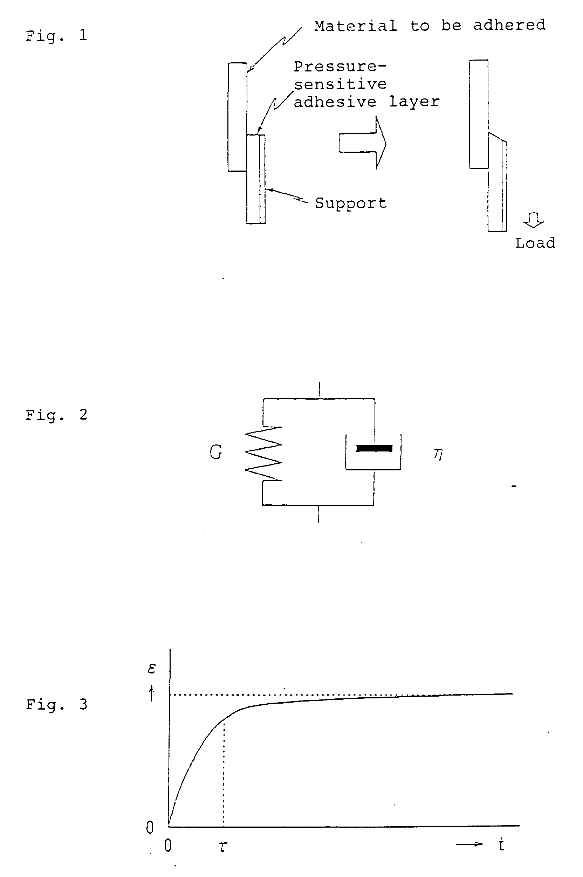

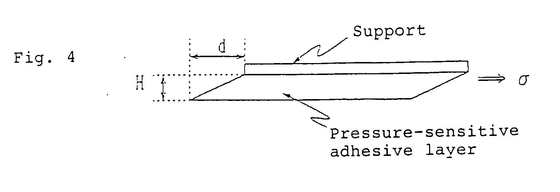

The Voigt model is a model shown by a parallel bonding of a spring as an elastic body and a dash pot moving in a liquid having a viscosity coefficient ($\eta$) as shown in Fig. 2, and is a model which is finally an elastic body when it is applied with an external force but showing a viscoelastic body showing a so-called deformation having a retarded elasticity, that is, showing that the increase of the deformation is retarded in time.

That is, as shown in Fig. 3, the strain ($\varepsilon$) to a definite stress is increased with time (t) but is gradually converged to a definite value. In the pressure-sensitive adhesive layer, the time until the strain is covered to a definite value is within 1,000 seconds.

The shearing elastic coefficient (G) and the retarding time ($\tau$) in the present invention are calculated from the sample deformed as shown in Fig. 4 by the test shown below.

That is, if the strain of the shearing creep at a time t is $\varepsilon$(t), in the Voigt model the strain becomes as shown in the following formula.

$$\varepsilon(t) = \sigma \cdot 1/G \cdot (1 - e^{-t/\tau}) \tag{1}$$

$\sigma$: Stress (dyn/cm$^2$)
G: Shearing elastic coefficient (dyn/cm$^2$)
$\tau$: Retarding time (second)
    or

$$\varepsilon = d/H$$

Since the deformation is finished at t = 1000, then

$$\varepsilon(\infty) \fallingdotseq \varepsilon(1000) = \sigma \cdot 1/G.$$

If H is assumed to be unchanged, $\varepsilon$ (1000) becomes as follows.

$$\varepsilon(1000) = d(t=1000)/H = \sigma \cdot 1/G \tag{2}$$

Next, if t = $\tau$, the formula 1 becomes as follows.

$$\varepsilon(\tau) = \sigma \cdot 1/G \cdot (1 - e^{-1}) \fallingdotseq \varepsilon(\infty) \times 0.632 \fallingdotseq \varepsilon(1000) \times 0.632$$

6

$$d(t=\tau) \fallingdotseq d(t=1000) \times 0.632 \qquad (4)$$

Thus, the shearing elastic coefficient (G) is obtained from formula 2 described above. Further, d at t=τ is obtained from formula 3, and retarding time (τ) can be presumed from the actually measured value.

In the medical adhesive tape of the present invention, the shearing elastic coefficient (G) of the pressure-sensitive adhesive layer obtained as described above is adjusted to be from 0.02 N/cm$^2$ ($2.0 \times 10^3$ to $6.0 \times 10^4$ dyn/cm$^2$,) and preferably from 0.03 N/cm$^2$ to 0.5 N/cm$^2$ ($3.0 \times 10^3$ to $5.0 \times 10^4$ dye/cm$^2$).

If the shearing elastic coefficient (G) is lower than 0.02 N/cm$^2$ to 0.6 N/cm$^2$ ($2.0 \times 10^3$ dyn/cm$^2$,) the cohesive force is insufficient and the pressure-sensitive adhesive layer is liable to be broken and even if the layer is not broken, the adhesion feeling becomes greatly poor. If the shearing elastic coefficient (G) is higher than 0.6 N/cm$^2$ ($6.0 \times 10^4$ dyn/cm$^2$,) the dispersion of stress at releasing the pressure-sensitive adhesive layer is insufficient to cause a physical stimulation, and since the amount of keratin released become large, whereby the objects of the present invention cannot be sufficiently attained and a delicate balance between the skin stimulative property and the skin adhesion is difficult to obtain.

On the other hand, it is necessary to control the retarding time (τ) to shorter than 30 seconds, and preferably shorter than 25 seconds. If the retarding time is longer than 30 seconds, a sufficient stress dispersion is not obtained as same as in the case of the G value and the effect aimed by the present invention cannot be sufficiently exhibited.

Furthermore, in the case of the tape preparation of the present invention containing percutaneous absorption-type drugs in the pressure-sensitive adhesive layer, when the drugs are contained in the pressure-sensitive adhesive layer in a crystal state, a filling effect causes, whereby the lower limit of the shearing elastic coefficient (G) becomes high a little and the G value becomes about 0.035 N/cm$^2$ ($3.5 \times 10^3$ dyn/cm$^2$) or higher.

Since the medical adhesive tape and the tape preparation of the present invention are designed such that the creep characteristics of the pressure-sensitive adhesive layer are adjusted to become lower than the specific initial shear modulus and also the permanent shearing strain formed after adding a shearing stress becomes lower than the specific value, the pressure-sensitive adhesive layer has a good skin adhesion and also can decrease the skin stimulation and a pain at releasing the pressure-sensitive adhesive layer from a skin surface. Thus, the medical adhesive tape and the tape preparation giving a soft feeling to a skin at adhering and releasing are obtained. In particular, when the pressure-sensitive adhesive layer is crosslinked and the pressure-sensitive adhesive layer coincides with the sinple Voigt model, the pressure-sensitive adhesive layer has the specific shearing modulus and retarding time, becomes excellent in the stress moderating property and the stress dispersing property, can decrease the skin stimulation and pain, and can keep a delicate balance between the skin adhesion and the skin stimulative property.

The present invention is explained in more detail by the following examples, wherein all parts and %, unless otherwise indicated, are by weight.

EXAMPLES 1 TO 10 AND COMPARATIVE EXAMPLES 1 TO 9

Each pressure-sensitive adhesive layer (thickness 60 μm) formed using each composition for pressure-sensitive adhesive layer shown in Table 1 and Table 2 was formed on the surface of a nonwoven fabric of a support (a laminate film composed of a polyester nonwoven fabric having a weight per unit area of 12 g/m$^2$ and a polyester film having a thickness of 2 μm) to obtain each medical adhesive tape or tape preparation.

In the Tables, % for the monomer shows the compounding ratio of each monomer, % for the crosslinking agent shows the proportion to solid components of the pressure-sensitive adhesive, and % for the plasticizer and the drug shows the proportion to the whole pressure-sensitive adhesive layer.

TABLE 1

| Example | Monomer (%) | Crosslinking Agent (%) | Plasticizer | Drug |
|---|---|---|---|---|
| 1 | 2-EHA/AA (95/5) | Colonate HL (0.15) | IPM (40%) | - |
| 2 | 2-EHA/AA (95/5) | Colonate HL (0.15) | IPM (50%) | - |
| 3 | 2-EHA/AA (95/5) | Colonate HL (0.15) | IPM (60%) | - |
| 4 | 2-EHA/AA (95/5) | Colonate HL (0.15) | OP (40%) | - |
| 5 | 2-EHA/AA (95/5) | Colonate HL (0.15) | IPM (40%) | ISDN (20%) |
| 6 | 2-EHA/AA (95/5) | ATAA (0.25) | IPM (40%) | ISDN (20%) |

TABLE 1 (continued)

| Example | Monomer (%) | Crosslinking Agent (%) | Plasticizer | Drug |
|---|---|---|---|---|
| 7 | 2-EHA/VP/AA (75/22/3) | Colonate HL (0.50) | IPM (50%) | - |
| 8 | 2-EHA/VP/AA (75/22/3) | Colonate HL (0.50) | IPM (50%) | ES (3%) |
| 9 | 2-EHA/VP/AA (75/22/3) | Colonate HL (0.60) | IPM/SC (45/5%) | - |
| 10 | 2-EHA/VP/AA (75/22/3) | Colonate HL (0.50) | IPM/OA (40/10%) | - |

Notes):
2-EHA = 2-Ethylhexyl acrylate
AA = Acrylic acid
VP = N-vinyl-2-pyrrolidone
IPM = Isopropyl myristate
OP = Octyl palmitate
ATAA = Aluminum triacetyl acetonate
SC = Sorbitan monocaprylate
OA = Oleic acid
Colonate HL = Trifunctional isocyanate (trade name, made by Nippon Polyurethane Co.)
ISDN = Isosorbid dinitrate
ES = Estradiol

TABLE 2

| Comparative Example | Monomer (%) | Crosslinking Agent (%) | Plasticizer | Drug |
|---|---|---|---|---|
| 1 | 2-EHA/AA (95/5) | - | IPM (50%) | - |
| 2 | 2-EHA/AA (95/5) | Colonate HL (0.15) | - | - |
| 3 | 2-EHA/AA (95/5) | - | - | ISDN (20%) |
| 4 | 2-EHA/AA (95/5) | Colonate HL (0.03) | IPM (50%) | - |
| 5 | 2-EHA/AA (95/5) | Colonate HL (0.15) | IPM (10%) | - |
| 6 | 2-EHA/AA (95/5) | Colonate HL (0.15) | IPM (90%) | - |
| 7 | 2-EHA/AA (95/5) | - | IPM (40%) | ISDN (20%) |
| 8 | 2-EHA/VP/AA (75/22/3) | - | - | - |
| 9 | 2-EHA/VP/AA (75/22/3) | ATAA (0.50) | OP (90%) | - |

Notes):
2-EHA = 2-Ethylhexyl acrylate
AA = acrylic acid
VP = N-Vinyl-2-pyrrolidone
IPM = Isopropyl myristate
OP = Octyl palmitate
ATAA = Aluminum triacetyl acetonate
Colonate HL = Trifunctional isocyanate
ISDN = Isosorbid dinitrate

On the medical adhesive tapes and the tape preparations obtained, the following characteristic evaluations were made. The results obtained are shown in Table 3 and Table 4.

[Shear modulus]

As shown in Fig. 1, each of the medical adhesive tapes or the tape preparations of the present invention was adhered to a bakelite plate such that the adhered area became 1 cm$^2$, and the adhered sample was closely adhered by reciprocating once a roller of 2 kg on the sample thus adhered.

After adhering the sample, from the shearing strain at applying a load of 50 g/cm$^2$ to the shearing direction for 30 seconds, the initial shear modulus was calculated according to the following formula.

$$E = W \cdot g/S \times d/X$$

E:     Shear modulus (dyn/cm$^2$)
W:     Weight (g) of the load
g:     Acceleration of gravity (cm/sec$^2$)
S:     Adhered area (cm$^2$)
d:     Thickness ($\mu$m) of the pressure-sensitive layer
X:     Shearing strain ($\mu$m).

[Permanent shearing strain]

The shear moved distance at applying the load for 30 minutes as described above was measured by a laser feed monitor (manufactured by Keyence Co.).

The proportion of the permanent shearing strain to the shearing strain at removing the load obtained above was calculated.

[Adhesive force]

Each strip sample cut into a width of 12 mm was adhered to a bakelite plate. After adhering the sample by reciprocating once a roller at a load of 850 g, the sample was peeled in the direction of 180° at a speed of 300 mm/minute, and the peeling force was measured.

[Skin stimulative property]

Each sample cut into a size of 5 cm$^2$ was applied to the inside of a brachium of each of 5 volunteers. After 8 hours since then, the sample was peeled. The skin stimulative property at peeling was obtained according to the following evaluation standard, and the average value thereof was calculated.

1: No skin stimulation
2: Slight skin stimulation
3: Small skin stimulation
4: Skin stimulation
5: Strong skin stimulation

[Adhering property]

In the above skin stimulative property test, the skin adhered state at peeling the sample was visually evaluated. In addition, the case that a rise on a considerable area was observed was evaluated as 1, the case that the sample was completely adhered was evaluated as 5, and the adhering property was evaluated by 5 stages.

[Adhesive remaining]

In the skin stimulative property test described above, after peeling each sample from the skin surface, the residue of the pressure-sensitive adhesive remained on the skin surface was visually observed. The case that the residue of the pressure-sensitive adhesive was observed on the whole adhered surface was evaluated as 1, the case that no residue of the pressure-sensitive adhesive was observed on the skin surface and no tackiness was observed was evaluated as 5, and the remaining state of the pressure-sensitive adhesive after peeling the sample was evaluated in 5 stages.

TABLE 3

| Example | (A) N/cm$^2$ (dyn/cm$^2$) | (B) (%) | (C) (g/12mm) | (D) | (E) | (F) |
|---|---|---|---|---|---|---|
| 1 | 0.144 (1.44×10$^4$) | 9.3 | 43 | 1.4 | 4.6 | 5.0 |
| 2 | 0.072 (7.17×10$^3$) | 12.3 | 40 | 1.0 | 5.0 | 5.0 |
| 3 | 0.046 (4.61×10$^3$) | 22.1 | 25 | 1.0 | 5.0 | 5.0 |
| 4 | 0.243 (2.43×10$^4$) | 7.8 | 39 | 1.4 | 4.6 | 5.0 |
| 5 | 0.285 (2.85×10$^4$) | 11.3 | 34 | 1.0 | 5.0 | 5.0 |
| 6 | 0.330 (3.30×10$^4$) | 8.3 | 31 | 1.2 | 5.0 | 5.0 |
| 7 | 0.206 (2.06×10$^4$) | 20.3 | 85 | 1.0 | 5.0 | 5.0 |
| 8 | 0.625 (6.25×10$^4$) | 12.0 | 82 | 1.0 | 5.0 | 5.0 |
| 9 | 0.233 (2.33×10$^4$) | 17.6 | 80 | 1.0 | 5.0 | 5.0 |
| 10 | 0.186 (1.86×10$^4$) | 19.3 | 88 | 1.0 | 5.0 | 5.0 |

(A): Shear modulus
(B): Permanent strain
(C): Adhesive force
(D): Skin stimulative property
(E): Adhering property
(F): Adhesive remaining

TABLE 4

| Comparative Example | (A) N/cm$^2$ (dyn/cm$^2$) | | (B) (%) | (C) (g/12mm) | (D) | (E) | (F) |
|---|---|---|---|---|---|---|---|
| 1 | - | - | - | - | - | - | - |
| 2 | 1.970 | (1.97×10$^5$) | 45.5 | 344 | 3.8 | 4.2 | 5.0 |
| 3 | 1.470 | (1.47×10$^5$) | 52.8 | 327 | 4.4 | 4.6 | 5.0 |
| 4 | 0.015 | (1.46×10$^3$) | 46.2 | 21 | 1.0 | 5.0 | 1.4 |
| 5 | 1.240 | (1.24×10$^5$) | 40.1 | 160 | 2.8 | 4.4 | 5.0 |
| 6 | - | - | - | - | - | - | - |
| 7 | - | - | - | - | - | - | - |
| 8 | 1.840 | (1.84×10$^5$) | 42.3 | 438 | 4.8 | 4.0 | 5.0 |
| 9 | - | - | - | - | - | - | - |

(A), (B), (C), (D), (E), and (F) are same as in Table 3.

<u>EXAMPLES 11 TO 18 AND COMPARATIVE EXAMPLES 10 TO 16</u>

Each pressure-sensitive adhesive layer (thickness 55 μm) formed by using each composition for the pressure-sensitive adhesive layer shown in Table 5 was formed on the nonwoven fabric surface of a support (laminate film composed of a polyester nonwoven fabric having a weight per unit area of 12 g/m$^2$ and a polyester film having a thickness of 2 μm) to obtain each of medical adhesive tapes and tape preparations. The units shown in Table 5 are the same as in Table 1 and Table 2.

TABLE 5

| Run No. | Monomer (%) | Crosslinking Agent (%) | Plasticizer | Drug |
|---|---|---|---|---|
| 1 | 2-EHA/AA (95/5) | - | - | - |
| 2 | 2-EHA/AA (95/5) | Colonate HL (0.063) | - | - |
| 3 | 2-EHA/AA (95/5) | Colonate HL (0.125) | - | - |
| 4 | 2-EHA/AA (95/5) | Colonate HL (0.125) | IPN (10%) | - |
| 5 | 2-EHA/AA (95/5) | Colonate HL (0.125) | IPM (20%) | - |
| 6 | 2-EHA/AA (95/5) | Colonate HL (0.125) | IPM (40%) | - |
| 7 | 2-EHA/AA (95/5) | Colonate HL (0.125) | IPM (60%) | - |
| 8 | 2-EHA/AA (95/5) | - | IPM (50%) | - |
| 9 | 2-EHA/AA (95/5) | Colonate HL (0.063) | IPM (50%) | - |
| 10 | 2-EHA/AA (95/5) | Colonate HL (0.125) | IPM (50%) | - |
| 11 | 2-EHA/AA (95/5) | Colonate HL (0.188) | IPM (50%) | - |
| 12 | 2-EHA/AA (95/5) | Colonate HL (0.125) | IPM (40%) | ISDN (17%) |
| 13 | 2-EFA/AA (95/5) | Colonate HL (0.125) | OP (40%) | TNG (10%) |
| 14 | 2-EHA/VP/AA (72/25/3) | Colonate HL (0.500) | IPM (50%) | ES (3%) |
| 15 | 2-EHA/VP/AA (72/25/3) | ATAA (1.000) | IPM (40%) | MP (10%) |

Notes):
2-EHA = 2-Ethylhexyl acetylate
AA = Acrylic acid
VP = N-Vinyl-2-pyrrolidone
IPM = Isopropyl Myristate
OP = Octyl palmitate
ATAA = Aluminum triacetyl acetate
Colonate HL = Trifunctional isocyanate (trade name, made by Nippon Polyurethane Co.)
ISDN = Isosorbid Dinitrate
TNG = Trinitroglycerol
ES = Estradiol
MP = Metprorol • free form
Run Nos. 1 to 5 = Comparative Examples 10 to 14.
Run Nos. 6 and 7 = Examples 11 and 12.
Run Nos. 8 and 9 = Comparative Examples 15 and 16.
Run Nos. 10 to 15 = Examples 13 to 18.

On each of the medical adhesive tapes or the tape preparations obtained, the following characteristic evaluations were made. The results obtained are shown in Tables 6 to 9.

[Viscoelastic characteristics]

Each sample was adhered to a bakelite plate with an adhered area of 1 cm × 1 cm and after closely adhering the sample by reciprocating once a roller of 2 kg, the assembly was allowed to stand for 30 minutes. By applying a shearing stress of a load of 50 g to the sample, the deformation amount of the sample to the stress was measured at a time interval of 5 seconds using a laser feed monitor. By calculating the measured result according to the calculation methods described above, $\varepsilon(t=1000)$, $\varepsilon(t=1000) \times (1-e^{-1})$, the G value, and the $\tau$ value were obtained.

[Adhesive force]

Each strip sample cut into a width of 12 mm was adhered to a bakelite plate, after closely adhering the sample by reciprocating a roller of 300 g, the sample was peeled in the direction of 180° at a speed of 300 mm/minute, and the peeling force was measured.

[Keratin released amount]

Each disk sample cut into a diameter of 16 mm was adhered to the inside of the brachium of each of 3 volunteers for 2 hours, after peeling the sample, the sample was immersed in a dye solution (Gentian violet 1%, Brilliant green 0.5%, distilled water 98.5%) for 3 minutes, and the sample was then washed with water and the keratin cell attached to the sample surface was dyed.

The sample was immersed in a 5% aqueous solution of sodium dodecylsulfate a whole day and night to extract the dyed solution and by measuring the absorbance (595 nm) of the extract, the number of the keratin cells released from the skin surface was compared. That is, it could be judged that as the absorbance was larger, the amount of the keratin released was larger.

In addition, between the number of the released keratin cells counted by a microscope and the above absorbance, a good correlation was confirmed.

[Pain]

Each sample was adhered to the inside of the brachium of each of 5 volunteers, after 2 hours since then, the sample was peeled, and the pain at peeling was measured. The pain was evaluated in 5 stages and the case of the smallest pain was defined as 1 and the average value was obtained. In addition, Sample No. 1 was defined 5 as the standard for the evaluation.

TABLE 6

| Time t (sec) | Deformed Amount d ($\mu$m) of Pressure-Sensitive Adhesive Layer | | | | | | | |
|---|---|---|---|---|---|---|---|---|
| | No. 1 | No. 2 | No. 3 | No. 4 | No. 5 | No. 6 | No. 7 | No. 8 |
| 0 | 0 | 0 | 0 | 0 | 0 | 0 | 0 | 0 |
| 5 | 13 | 17 | 17 | 17 | 39 | 137 | 584 | 321 |
| 10 | 20 | 17 | 19 | 20 | 43 | 189 | 655 | 1260 |
| 15 | 20 | 17 | 20 | 23 | 46 | 208 | 678 | 2315 |
| 20 | 23 | 21 | 20 | 23 | 48 | 221 | 691 | 5612 |
| 25 | 23 | 21 | 20 | 26 | 51 | 229 | 701 | broken |
| 30 | 23 | 24 | 21 | 26 | 52 | 235 | 707 | |
| 35 | 23 | 24 | 23 | 26 | 56 | 241 | 714 | |
| 40 | 26 | 24 | 23 | 28 | 59 | 247 | 717 | |
| 45 | 26 | 24 | 23 | 28 | 59 | 247 | 721 | |
| 50 | 26 | 27 | 23 | 28 | 62 | 254 | 726 | |
| 55 | 26 | 27 | 23 | 29 | 62 | 254 | 727 | |
| 60 | 26 | 27 | 23 | 29 | 62 | 257 | 729 | |
| 65 | 30 | 27 | 23 | 30 | 65 | 260 | 730 | |
| 70 | 30 | 27 | 23 | 32 | 66 | 261 | 731 | |
| 75 | 30 | 27 | 23 | 33 | 66 | 264 | 731 | |
| 80 | 30 | 27 | 23 | 33 | 69 | 267 | 731 | |
| 85 | 30 | 28 | 23 | 33 | 70 | 267 | 731 | |

TABLE 6 (continued)

| Time t (sec) | Deformed Amount d (μm) of Pressure-Sensitive Adhesive Layer | | | | | | | |
|---|---|---|---|---|---|---|---|---|
| | No. 1 | No. 2 | No. 3 | No. 4 | No. 5 | No. 6 | No. 7 | No. 8 |
| 90 | 30 | 28 | 23 | 33 | 72 | 267 | 731 | |
| 95 | 30 | 28 | 23 | 33 | 74 | 270 | 731 | |
| 100 | 33 | 28 | 23 | 34 | 76 | 270 | 732 | |
| 105 | 33 | 28 | 23 | 34 | 77 | 270 | 731 | |
| 110 | 33 | 28 | 23 | 34 | 77 | 273 | 731 | |
| 115 | 33 | 28 | 23 | 34 | 78 | 273 | 731 | |
| 120 | 33 | 29 | 23 | 35 | 79 | 273 | 731 | |
| 125 | 33 | 30 | 24 | 35 | 80 | 277 | 731 | |
| 130 | 33 | 30 | 24 | 37 | 82 | 277 | 731 | |
| 150 | 34 | 30 | 24 | 38 | 82 | 280 | 731 | |
| 200 | 35 | 30 | 25 | 40 | 86 | 285 | 731 | |
| 250 | 36 | 34 | 26 | 43 | 87 | 286 | 732 | |
| 500 | 39 | 40 | 32 | 49 | 87 | 285 | 731 | |
| 750 | 44 | 47 | 33 | 50 | 87 | 285 | 731 | |
| 1000 | 48 | 47 | 33 | 49 | 87 | 285 | 731 | |

TABLE 7

| Time t (sec) | Deformed Amount d (μm) of Pressure-Sensitive Adhesive Layer | | | | | | |
|---|---|---|---|---|---|---|---|
| | No. 9 | No. 10 | No. 11 | No. 12 | No. 13 | No. 14 | No. 15 |
| 0 | 0 | 0 | 0 | 0 | 0 | 0 | 0 |
| 5 | 449 | 222 | 173 | 87 | 26 | 40 | 52 |
| 10 | 933 | 359 | 221 | 100 | 537 | 52 | 55 |
| 15 | 1105 | 401 | 234 | 108 | 572 | 52 | 58 |
| 20 | 1199 | 424 | 241 | 113 | 585 | 52 | 58 |
| 25 | 1261 | 437 | 245 | 116 | 592 | 52 | 58 |
| 30 | 1310 | 449 | 251 | 119 | 595 | 52 | 58 |
| 35 | 1346 | 456 | 254 | 122 | 602 | 52 | 59 |
| 40 | 1376 | 463 | 254 | 126 | 605 | 52 | 59 |
| 45 | 1401 | 469 | 257 | 126 | 607 | 52 | 59 |
| 50 | 1421 | 476 | 260 | 126 | 608 | 53 | 59 |
| 55 | 1440 | 479 | 260 | 128 | 611 | 56 | 60 |
| 60 | 1456 | 482 | 260 | 129 | 613 | 55 | 60 |
| 65 | 1472 | 485 | 263 | 132 | 615 | 56 | 60 |
| 70 | 1486 | 489 | 264 | 132 | 615 | 56 | 60 |
| 75 | 1499 | 492 | 264 | 135 | 618 | 56 | 60 |

TABLE 7 (continued)

| Time t (sec) | Deformed Amount d (μm) of Pressure-Sensitive Adhesive Layer | | | | | | |
|---|---|---|---|---|---|---|---|
| | No. 9 | No. 10 | No. 11 | No. 12 | No. 13 | No. 14 | No. 15 |
| 80 | 1508 | 494 | 264 | 135 | 618 | 56 | 62 |
| 85 | 1518 | 498 | 264 | 135 | 618 | 56 | 62 |
| 90 | 1528 | 502 | 267 | 135 | 621 | 56 | 62 |
| 95 | 1538 | 502 | 267 | 139 | 621 | 56 | 62 |
| 100 | 1544 | 505 | 267 | 139 | 621 | 56 | 64 |
| 105 | 1554 | 508 | 270 | 139 | 621 | 56 | 64 |
| 110 | 1560 | 509 | 270 | 139 | 624 | 57 | 64 |
| 115 | 1570 | 511 | 270 | 140 | 624 | 57 | 64 |
| 120 | 1573 | 515 | 270 | 140 | 624 | 58 | 67 |
| 125 | 1583 | 518 | 271 | 141 | 624 | 58 | 62 |
| 130 | 1595 | 520 | 271 | 141 | 624 | 59 | 62 |
| 150 | 1621 | 528 | 271 | 142 | 628 | 59 | 69 |
| 200 | 1680 | 546 | 272 | 144 | 631 | 62 | 71 |
| 250 | 1697 | 553 | 271 | 144 | 634 | 66 | 73 |
| 500 | 1698 | 553 | 271 | 144 | 634 | 69 | 74 |
| 750 | 1697 | 553 | 271 | 144 | 634 | 69 | 74 |
| 1000 | 1697 | 553 | 271 | 144 | 634 | 69 | 74 |

TABLE 8

| | Sample No. | | | | | | | |
|---|---|---|---|---|---|---|---|---|
| | No. 1 | No. 2 | No. 3 | No. 4 | No. 5 | No. 6 | No. 7 | No. 8 |
| $\varepsilon(t=1000)$ (μm) | - | 47 | 33 | 49 | 87 | 285 | 731 | - |
| $\varepsilon(t=1000) \times (1-e^{-1})$ (μm) | - | 30 | 209 | 31 | 55 | 180 | 462 | - |
| G N/cm$^2$ (dyn/cm$^2$) | - | 0.573 (5.73 $\times 10^4$) | 0.817 (8.17 $\times 10^4$) | 0.550 (5.50 $\times 10^4$) | 0.310 (3.10 $\times 10^4$) | 0.095 (9.49 $\times 10^3$) | 0.037 (3.69 $\times 10^3$) | - |
| $\tau$ (sec) | - | 120-125 | 25-30 | 65-70 | 30-35 | 5-10 | 0-5 | - |
| Adhesive force (g/12mm) | 351 | 362 | 361 | 415 | 114 | 92 | 70 | - |
| Released amount of keratin (Abs.) | 1.40 | 1.54 | 1.51 | 1.31 | 0.52 | 0.39 | 0.41 | - |
| Pain | 5 | 5 | 5 | 5 | 3 | 1 | 1 | - |

TABLE 9

| | Sample No. | | | | | | |
|---|---|---|---|---|---|---|---|
| | No. 9 | No. 10 | No. 11 | No. 12 | No. 13 | No. 14 | No. 15 |
| $\varepsilon(t=1000)$ ($\mu$m) | 1697 | 553 | 271 | 144 | 634 | 69 | 74 |
| $\varepsilon(t=1000) \times (1-e^{-1})$ ($\mu$m) | 1073 | 349 | 171 | 91 | 401 | 44 | 47 |
| G N/cm$^2$ (dyn/cm$^2$) | 0.016 (1.59 $\times 10^3$) | 0.049 (4.87 $\times 10^3$) | 0.099 (9.94 $\times 10^3$) | 0.187 (1.87 $\times 10^4$) | 0.043 (4.25 $\times 10^3$) | 0.391 (3.91 $\times 10^4$) | 0.397 (3.97 $\times 10^4$) |
| $\tau$ (sec) | 10-15 | 5-10 | 0-5 | 5-10 | 5-10 | 5-10 | 0-5 |
| Adhesive force (g/12mm) | 67 | 69 | 74 | 68 | 61 | 95 | 68 |
| Released amount of keratin (Abs.) | 0.34 | 0.30 | 0.29 | 0.36 | 0.25 | 0.60 | 0.42 |
| Pain | 2 | 1 | 1 | 1 | 1 | 2 | 1 |

In the results shown in Table 8 and Table 9 above, in the medical adhesive tape of Sample No. 1 (Comparative Example 10). the deformation was not finished and hence the value G and the value $\tau$ could not be obtained.

Further, in the medical adhesive tape of Sample No. 8 (Comparative Example 15), the cohesive failure occurred and sample could not be tested.

Furthermore, in the medical adhesive tape of Sample No. 8 (Comparative Example 16), the sample showed excellent values in the adhesive force, the released amount of the keratin, and the pain, and also the cohesive failure did not occur but the sample showed a tacky feeling, had a feeling of requiring a care for falling off of the sample, the actual tacky feeling was bad, and the sample was unsuitable for a practical use.

The G values and the $\tau$ values of the medical adhesive tapes of Sample Nos. 6, 7, 10 and 11 and the tape preparations of Sample Nos. 12 to 15 were within the present invention (Examples 11 to 18) and these samples were excellent for practical use.

The medical adhesive tape of Sample No. 5 (Comparative Example 14) could be practically used but the sample gave some pain at peeling and hence was not particularly excellent one.

**Claims**

1. A medical adhesive tape comprising a flexible support having formed on at least one surface thereof a hydrophobic pressure-sensitive adhesive layer, wherein when the pressure-sensitive adhesive layer is adhered to a bakelite plate, an initial shear modulus at applying a load of 50 g/cm$^2$ to a shearing direction is 1 N/cm$^2$ ($1 \times 10^5$ dyn/cm$^2$) or less and the permanent shearing strain occurring in the pressure-sensitive adhesive layer at removing the load after adding the load for 30 minutes is 30% or less of the shearing strain at removing the load.

2. A medical adhesive tape of claim 1, wherein the pressure-sensitive adhesive layer comprises a copolymer obtained by copolymerizing a (meth)acrylic acid alkyl ester having an alkyl group having an average carbon atom number of from 4 to 18 as the main component monomer.

3. A medical adhesive tape of claim 1, wherein the pressure-sensitive adhesive layer contains an organic liquid material in a compatible state.

4. A medical adhesive tape of claim 3, wherein the organic liquid material is at least one liquid material selected-from the group of consisting of fatty acid esters, fatty acids, and surface active agents.

5. A medical adhesive tape of claim 1, wherein the pressure-sensitive adhesive layer is a crosslinked structural material.

6. A medical adhesive tape of claim 5, wherein the deformation strain of the pressure-sensitive adhesive layer at adding a shearing stress to the layer has a shearing elastic coefficient (G) and a retarding time ($\tau$) satisfying the following values in the case of applying it to a simple Voigt model;

$$0.6 \text{ N/cm}^2 > G > 0.02 \text{ N/cm}^2 \, [6.0 \times 10^4 > G > 2.0 \times 10^3 \, (\text{dyn/cm}^2)] \, 30 > \tau \text{ (sec.)}$$

7. A tape preparation comprising a flexible support having formed on at least one surface thereof a hydrophobic pressure-sensitive adhesive layer, wherein when the pressure-sensitive adhesive layer is adhered to a bakelite plate, the initial shear modulus at applying a load of 50 g/cm$^2$ to the shearing direction for 30 seconds is 1 N/cm$^2$ ($1 \times 10^5$ dyn/cm$^2$) or less, the permanent shearing strain occurring in the pressure-sensitive layer at removing the load after adding the load for 30 minutes is 30% or less of the shearing strain at removing the load, and the pressure-sensitive adhesive layer contains percutaneous absorption-type drugs.

## Patentansprüche

1. Medizinisches Heftpflaster, das einen flexiblen Träger aufweist, bei dem auf mindestens einer Oberfläche eine hydrophobe druckempfindliche Klebschicht ausgebildet ist, wobei beim Kleben der druckempfindlichen Klebschicht an eine Bakelit-Platte bei Anwendung einer Belastung von 50 g/cm$^2$ in einer Scherrichtung der anfängliche Schermodul 1 N/cm$^2$ ($1 \times 10^5$ dyn/cm$^2$) oder weniger beträgt und die in der druckempfindlichen Klebschicht auftretende Dauerscherverformung bei der Entfernung der Belastung nach 30-minütiger Anwendung der Belastung 30 % oder weniger der Scherverformung bei der Entfernung der Belastung beträgt.

2. Medizinisches Heftpflaster nach Anspruch 1, wobei die druckempfindliche Klebschicht ein Copolymer aufweist, das durch Copolymerisation eines (Meth)acrylsäurealkylesters mit einer Alkyl-Gruppe mit einer durchschnittlichen Kohlenstoffanzahl von 4 bis 18 als Hauptmonomer-Komponente erhalten wurde.

3. Medizinisches Heftpflaster nach Anspruch 1, wobei die druckempfindliche Klebschicht eine organische Flüssigkeit in einem verträglichen Zustand enthält.

4. Medizinisches Heftpflaster nach Anspruch 3, wobei die organische Flüssigkeit mindestens eine aus der aus Fettsäureestern, Fettsäuren und oberflächenaktiven Mitteln bestehenden Gruppe ausgewählte Flüssigkeit ist.

5. Medizinisches Heftpflaster nach Anspruch 1, wobei die druckempfindliche Klebschicht ein Material mit vernetzter Struktur ist.

6. Medizinisches Heftpflaster nach Anspruch 5, wobei die Deformationsbeanspruchung der druckempfindlichen Klebschicht beim Anlegen von Scherspannung an die Schicht einen Scherelastizitätskoeffizienten (G) und eine Verzögerungszeit ($\tau$) hat, die im Falle der Anwendung auf ein einfaches Voigt-Modell die folgenden Werte erfüllen:

$$0,6 \text{ N/cm}^2 > G > 0,02 \text{ N/cm}^2 \, [6,0 \times 10^4 > G > 2,0 \times 10^3 \, (\text{dyn/cm}^2)] \, 30 > \tau \text{ (s)}$$

7. Pflasterpräparat, das einen flexiblen Träger aufweist, bei dem auf mindestens einer Oberfläche eine hydrophobe druckempfindliche Klebschicht ausgebildet ist, wobei beim Kleben der druckempfindlichen Klebschicht an eine Bakelit-Platte bei 30-sekündiger Anwendung einer Belastung von 50 g/cm$^2$ zur Scherrichtung der anfängliche Schermodul 1 N/cm$^2$ ($1 \times 10^5$ dyn/cm$^2$) oder weniger beträgt und die in der druckempfindlichen Klebschicht auftretende Dauerscherverformung bei der Entfernung der Belastung nach 30-minütiger Anwendung der Belastung 30 % oder weniger der Scherverformung bei der Entfernung der Belastung beträgt und die druckempfindliche Klebschicht perkutan absorbierbare Arzneimittel enthält.

## Revendications

1. Ruban adhésif médical comprenant un support flexible ayant une couche d'adhésif hydrophobe sensible à la pression formée sur au moins une surface de celui-ci, caractérisé en ce que quand la couche d'adhésif sensible à la pression est collée à une plaque de bakélite, un module de cisaillement initial en appliquant une charge de 50 g/cm$^2$ selon une direction de cisaillement est égal à 1 N/cm$^2$ ($1 \times 10^5$ dynes/cm$^2$) ou moins et en ce que la contrainte de cisaillement permanente se produisant dans la couche d'adhésif sensible à la pression en enlevant la charge après addition de la charge pendant 30 minutes est égale à 30 % ou moins de la contrainte de cisaillement en enlevant la charge.

2. Ruban adhésif médical selon la revendication 1, caractérisé en ce que la couche d'adhésif sensible à la pression comprend un copolymère obtenu par copolymérisation d'un ester alkylique d'acide (méth)acrylique ayant un groupe alkyle comportant un nombre moyen d'atomes de carbone de 4 à 18 comme monomère composant principal.

3. Ruban adhésif médical selon la revendication 1, caractérisé en ce que la couche d'adhésif sensible à la pression contient un matériau liquide organique dans un état compatible.

4. Ruban adhésif médical selon la revendication 3, caractérisé en ce que le matériau liquide organique est au moins un matériau liquide choisi dans le groupe constitué par les esters d'acides gras, les acides gras, et les agents tensioactifs.

5. Ruban adhésif médical selon la revendication 1, caractérisé en ce que la couche d'adhésif sensible à la pression est un matériau réticulé structurellement.

6. Ruban adhésif médical selon la revendication 5, caractérisé en ce que la contrainte de déformation de la couche d'adhésif sensible à la pression à l'addition d'une contrainte de cisaillement à la couche a un coefficient élastique de cisaillement (G) et un temps de retardement ($\tau$) satisfaisant les valeurs suivantes dans le cas où on l'applique à un modèle de Voigt simple :

$$0,6 \text{ N/cm}^2 > G > 0,02 \text{ N/cm}^2 \ [6,0 \times 10^4 > G > 2,0 \times 10^3 \ (\text{dyne/cm}^2)] \ 30 > \tau \ (s)$$

7. Préparation de ruban comprenant un support flexible ayant une couche d'adhésif hydrophobe sensible à la pression formée sur au moins une surface de celui-ci, caractérisé en ce que quand la couche d'adhésif sensible à la pression est collée à une plaque de bakélite, le module de cisaillement initial en appliquant une charge de 50 g/cm$^2$ selon une direction de cisaillement pendant 30 secondes est égal à 1 N/cm$^2$ (1 x 10$^5$ dynes/cm$^2$) ou moins, en ce que la contrainte de cisaillement permanente se produisant dans la couche d'adhésif sensible à la pression en enlevant la charge après addition de la charge pendant 30 minutes est égale à 30 % ou moins de la contrainte de cisaillement en enlevant la charge, et en ce que la couche d'adhésif sensible à la pression contient des médicaments du genre absorption percutanée.

Fig. 1

Material to be adhered

Pressure-
sensitive
adhesive layer

Support

Load

Fig. 2

G      η

Fig. 3

$\varepsilon$

0

0      $\tau$      $t$

Fig. 4

Support

Pressure-sensitive
adhesive layer

$d$

$H$

$\Rightarrow \sigma$